# EUROPEAN PATENT APPLICATION

(11) **EP 4 000 613 A1**
(43) Date of publication of application: **25.05.2022**
(21) Application number: 20020545.8
(22) Date of filing: 19.11.2020
(51) Int. Cl.: A61K 31/337, A61K 38/12, A61K 45/06, A61P 35/00, C07K 7/00

(54) **PHARMACEUTICAL COMBINATIONS COMPRISING A PEPTIDE CXCR4 INHIBITOR AND A TAXANE FOR TREATING CANCER**

(71) Applicant: Polyphor AG, 4123 Allschwil (CH)
(72) Inventor: Zimmermann, Johann, 79379 Mühlheim (DE)

(57) **Abstract**

The present invention relates to pharmaceutical combinations comprising a peptidic CXCR4 inhibitor and a taxane for use in a method for the prevention, delay of progression or treatment of cancer.

## Description

### Field of the invention

The present invention relates to pharmaceutical combinations comprising a peptidic CXCR4 inhibitor and a taxane and their use in a method for the prevention, delay of progression or treatment of cancer in a subject.

### Background of the invention

Despite the ever increasing number of cancer therapies in general, and combination cancer therapies in particular, cancer is still the third most common cause of death worldwide after cardiovascular diseases and infectious/parasitic diseases; in absolute numbers, this corresponds to 7.6 million deaths (ca. 13% of all deaths) in any given year. The WHO estimates deaths due to cancer to increase to 13.1 million by 2030, while the American Cancer Society expects over 1,685,210 new cancer cases diagnosed and 595,690 cancer deaths in the US in 2016.

Chemotherapy interferes with cell replication or cell metabolism. Typical chemotherapeutic agents include alkylating agents, nucleotide analogues such as gemcitabine and capecitabine, platinum agents such as cisplatin or oxaliplatin, topoisomerase I inhibitors such as campthotecin or irinotecan, topoisomerase II inhibitors such doxorubicin or mitoxanthrone, vinca alkaloids such as vinorelbine, and modulators of tubulins such as taxanes (I. Ojima et al. Exp. Opin. Ther. Patents 2016, 26, 1-20) and eribulin (U. Swami et al. Mar. Drugs 2015, 13, 5016-5058). Chemotherapy can be effective, however, often there are severe side effects, e.g., vomiting, low white blood cells (WBC), loss of hair, loss of weight and other toxic effects. Because of the extremely toxic side effects, many cancer patients cannot successfully finish a complete chemotherapy therapy. Chemotherapy-induced side effects have a significant impact on the quality of life of patients affected by cancer and may dramatically influence individual compliance with treatment. Adverse side effects associated with chemotherapeutic agents are in many cases the major dose-limiting toxicity (DLT) in the administration of these drugs.

Emerging tumor resistance during or after chemotherapy (N. Vasan et al. Nature 2019, 575, 299-309) and/or inadequate dosing due to dose-limitations in case of pre-existing or acquired resistance are additional serious limitations of chemotherapeutic treatment. Combination therapy of two chemotherapeutics with different mechanism of action can alleviate the resistance problem only to a certain extent. For all these reasons there is a great need for effective treatments of cancer.

Administration of two or more drugs to treat a given condition, such as cancer, generally raises a number of potential problems due to complex *in vivo* interactions between drugs. The effects of any single drug are related to its absorption, distribution, and elimination. When two drugs are introduced into the body, each drug can affect the absorption, distribution, and elimination of the other and hence, alter the effects of the other. For instance, one drug may inhibit, activate or induce the production of enzymes involved in a metabolic route of elimination of the other drug. Thus, when two drugs are administered to treat the same condition, it is unpredictable whether each will complement, have no effect on, or interfere with, the therapeutic activity of the other in a subject. Not only may the interaction between two drugs affect the intended therapeutic activity of each drug, but the interaction may increase the levels of toxic metabolites. The interaction may also heighten or lessen the side effects of each drug. Hence, upon administration of two drugs to treat a disease, it is unpredictable what change, either deterioration or improvement, will occur in the side effect profile of each drug. Additionally, it is difficult to accurately predict when the effects of the interaction between the two drugs will become manifest. For example, metabolic interactions between drugs may become apparent upon the initial administration of the second drug, after the two have reached a steady-state concentration or upon discontinuation of one of the drugs. Therefore, the effects of a combination therapy of two or more drugs cannot be easily predicted. Nevertheless, there is a distinct need for new treatment modalities such as useful combination therapies for the treatment of cancer.

### Summary of the invention

It has now unexpectedly been found that a combination comprising a peptidic CXCR4 inhibitor and a taxane is useful for the prevention, delay of progression or treatment of cancer, in particular breast cancer, metastatic breast cancer, and relapsed metastatic breast cancer. In a standard model established in cancer research, it was unexpectedly found that treatment with said combination provides an increased anti-tumor effect above the effect of either agent alone.

Taking these unexpected findings into account, the inventors herewith provide the present invention in its following aspects.

In a first aspect the present invention provides a pharmaceutical combination comprising:
(a) a peptidic CXCR4 inhibitor;
(b) a taxane; and
(c) optionally one or more pharmaceutically acceptable diluents, excipients or carriers.
In a second aspect the present invention provides a pharmaceutical combination as described herein, for use as a medicament.

In a third aspect the present invention provides a pharmaceutical combination as described herein, for use in a method for the prevention, delay of progression or treatment of cancer in a subject.

In a fourth aspect the present invention provides kit of parts comprising a first container, a second container and a package insert, wherein the first container comprises at least one dose of a medicament comprising a peptidic CXCR4 inhibitor; the second container comprises at least one dose of a medicament comprising a taxane, and the package insert comprises optionally instructions for treating a subject for cancer using the medicaments.

### Brief description of the figures

**Fig. 1****:** Timely events of the study. The experiment started with arrival and radiation of NOGEXL mice and was completed after 183 days.
**Fig. 2****:** Tumor growth of Ma15169 in humanized NOG-EXL mice and response to 10mg/kg Paclitaxel and 20mg/kg Balixafortide therapy. Treatment started at day 154 of the study (32 days after PDX implantation).

### Detailed description of the invention

For the purposes of interpreting this specification, the following definitions will apply and whenever appropriate, terms used in the singular will also include the plural and vice versa. It is to be understood that the terminology used herein is for the purpose of describing particular embodiments only and is not intended to be limiting. The terms "comprising", "having", and "including" are to be construed as open-ended terms (i.e. meaning "including, but not limited to,") unless otherwise noted.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The terms "individual," "subject" or "patient" are used herein interchangeably. In certain embodiments, the subject is a mammal. Mammals include, but are not limited to primates (including human and non-human primates). In a preferred embodiment, the subject is a human.

The term "pharmaceutically acceptable diluents, excipients or carriers" as used herein refers to diluents, excipients or carriers that are suitable for use with humans and/or animals without undue adverse side effects (such as toxicity, irritation, and allergic response) commensurate with a reasonable benefit/risk ratio. "Diluents" are agents which are added to the bulk volume of the active agent making up the solid composition. As a result, the size of the solid composition increases, which makes it easier to handle. Diluents are convenient when the dose of drug per solid composition is low and the solid composition would otherwise be too small. "Excipients" can be binders, lubricants, glidants, coating additives or combinations thereof. Thus, excipients are intended to serve multiple purposes. "Carriers" can be solvents, suspending agents or vehicles, for delivering the instant compounds to a subject.

The term "dose" as used herein refers to the total amount of an active ingredient (e.g., the peptidic CXCR4 inhibitor or taxane to be taken each time by a subject (e.g. a human).

The term "objective response rate" (ORR) as used herein refers to the proportion of patients with tumor size reduction of a predefined amount and for a minimum time period. Response duration usually is measured from the time of initial response until documented tumor progression. Generally, the FDA has defined ORR as the sum of partial responses plus complete responses. When defined in this manner, ORR is a direct measure of drug antitumor activity, which can be evaluated in a single-arm study. The ORR refers to the sum of complete response (CR) and partial response (PR).

The term "clinical benefit rate" (CBR) as used herein refers to the sum of complete response (CR), partial response (PR) and stable disease (SD) ≥6 months.

The term "complete response" (CR) as used herein in relation to target lesions refers to disappearance of all target lesions. Any pathological lymph nodes (whether target or non-target) must have reduction in short axis to <10 mm. The term complete response (CR) as used herein in relation to non-target lesions refers to disappearance of all non-target lesions and normalization of tumor marker level. All lymph nodes must be non-pathological in size (<10 mm short axis).

The term "partial response" (PR) as used herein in relation to target lesions refers to at least a 30% decrease in the sum of the diameters of target lesions, taking as reference the baseline sum diameters.

The term "progressive disease" (PD) as used herein in relation to target lesions refers to at least a 20% increase in the sum of the diameters of target lesions, taking as reference the smallest sum on study (this includes the baseline sum if that is the smallest on study). In addition to the relative increase of 20%, the sum must also demonstrate an absolute increase of at least 5 mm. The appearance of one or more new lesions is also considered progressions. The term progressive disease (PD) as used herein in relation to non-target lesions refers to appearance of one or more new lesions and/or unequivocal progression of existing non-target lesions. Unequivocal progression should not normally trump target lesion status. It must be representative of overall disease status change, not a single lesion increase.

The term "stable disease" (SD) as used herein in relation to target lesions refers to neither sufficient shrinkage to qualify for PR nor sufficient increase to qualify for PD, taking as reference the smallest sum diameters while on study.

The term "progression-free survival" (PFS) as used herein relates to the duration of time from start of treatment to time of progression or death, whichever occurs first.

The terms "cancer" and "cancerous" as used herein refer to or describe the physiological condition in mammals that is typically characterized by unregulated cell growth. A "tumor" comprises one or more cancerous cells. Examples of cancer include, but are not limited to, kaposi sarcoma, endometrial cancer, head and neck cancer, oesophageol cancer, breast cancers, lung cancer, pancreatic cancer, prostate cancer, colon cancer, ovarian cancer, and gastric cancer.

The term "metastatic cancer" as used herein means the state of cancer, e.g. the state of breast cancer where the cancer cells are transmitted from the original site to one or more sites elsewhere in the body, by the blood vessels or lymphatics, to form one or more secondary tumors at one or more sites or organs besides the original site or organ.

The term "solid tumor" or "solid tumor indication" used herein refers to an abnormal mass of tissue that usually does not contain cysts or liquid areas. Solid tumors may be benign (not cancer), or malignant (cancer). Preferably malignant solid tumors are treated with the methods of the present invention. Different types of malignant solid tumors are usually named for the type of cells that form them. Examples of malignant solid tumors are sarcomas, carcinomas, and lymphomas. Leukemias (cancers of the blood) generally do not form malignant solid tumors (definition according to the national cancer institute of the NIH). Malignant solid tumors include, but are not limited to, abnormal mass of cells which may stem from different tissue types.

The term "pharmaceutically acceptable salt" of a compound means a salt that is pharmaceutically acceptable and that it possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e. g. an alkaline metal ion, an alkaline earth metal ion, or an aluminum ion; or coordinates with an organic base such as ethanolamine, diethanolamine, triethanolamine, tromethamine, N-methylglucamine, and the like.

The term "about" as used herein refers to +/- 10% of a given measurement.

Thus, in a first aspect the present invention provides a pharmaceutical combination comprising:
(a) a peptidic CXCR4 inhibitor;
b) a taxane ; and
(c) optionally one or more pharmaceutically acceptable diluents, excipients or carriers.

### Peptidic CXCR4 inhibitors

The term "peptidic CXCR4 inhibitor" as used herein refers to a compound that binds to the CXCR4 receptor and generally antagonizes ligand (CXCL12)-induced signaling and can also act as an inverse agonist or a partial agonist of the CXCR4 receptor (W. Zhang et al. 2002, 27, 24515-24521).

In one embodiment the peptidic CXCR4 inhibitor is a backbone cyclized peptidic CXCR4 inhibitor.

In a further embodiment the peptidic CXCR4 inhibitor is a backbone cyclized peptidic compound, built up from 16 amino acid residues.

In a preferred embodiment the peptidic CXCR4 inhibitor is a backbone cyclized peptidic compound, built up from 16 amino acid residues, or pharmaceutically acceptable salts thereof, of the formula

cyclo(-Tyr¹-His²-Xaa³-Cys⁴-Ser⁵-Ala⁶-Xaa⁷-Xaa⁸-Arg⁹-Tyr¹⁰-Cys¹¹-Tyr¹²-Gln¹³-Xaa¹⁴-Xaa¹⁵-Pro¹⁶-) (I),

in which
Xaa³ is Ala; Tyr; or Tyr(Me);
Xaa⁷ is ^{D}Pro ; ^{D}Tyr; or ^{D}Tyr(Me);
Xaa⁸ is Dab; or Orn(iPr);
Xaa¹⁴ is Lys; or Lys(iPr);
Xaa¹⁵ is ^{D}Pro; or ^{D}Lys(iPr);
wherein
Tyr(Me) is (2S)-2-amino-3-(4-methoxyphenyl)-propanoic acid;
^{D}Tyr(Me) is (2R)-2-amino-3-(4-methoxyphenyl)-propanoic acid;
Dab is (2S)-2,4-diaminobutyric acid;
Orn(iPr) is (2*S*)-N^{ω}-isopropyl-2,5-diaminopentanoic acid;
Lys(iPr) is (2*S*)-N^{ω}-isopropyl-2,6-diaminohexanoic acid;
^{D}Lys(iPr) is (2*R*)-N^{ω}-isopropyl-2,6-diaminohexanoic acid;
wherein all of the amino acid residues, which are not explicitly designated as D-amino acid residues, are L-amino acid residues, and wherein the peptidic CXCR4 inhibitor has a disulfide bond between Cys⁴ and Cys¹¹.

In a more preferred embodiment the peptidic CXCR4 inhibitor is a backbone cyclized peptidic compound, built up from 16 amino acid residues, or pharmaceutically acceptable salts thereof, selected from the group consisting of cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala -^{D}Pro-Orn(iPr)-Arg-Tyr-Cys-Tyr-Gln-Lys(iPr)-^{D}Lys(iPr)-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr-Dab-Arg-Tyr-Cys-Tyr-Gln Lys(iPr)-^{D}Pro-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr -Cys-Ser-Ala-^{D}Tyr-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹,
or pharmaceutically acceptable salts thereof.

In an even more preferred embodiment the peptidic CXCR4 inhibitor is selected from the group consisting of cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, and cyclo(-Tyr-His-Tyr-Cys-Ser-Ala-^{D}Pro-Dab-Arg-Tyr-Cys-Tyr-Gln-Lys-^{D}Pro-Pro-) having a disulfide bond between Cys⁴ and Cys¹¹, or pharmaceutically acceptable salts thereof.

In a particular preferred embodiment the peptidic CXCR4 inhibitor is cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, or pharmaceutically acceptable sals thereof. Cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹ is also referred as POL6326 herein or balixafortide. POL6326 is a cyclic synthetic peptide consisting of 16 amino acids and an antagonist of the highly conserved chemokine receptor CXCR4. In vitro receptor binding studies demonstrated a significant affinity of POL6326 for the human CXCR4 receptor, as well as a general lack of significant binding to other potential target receptors.

Particularly suitable pharmaceutically acceptable salts of the peptidic CXCR4 inhibitor to be useful in the context of the present invention include the acetates, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonate, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid. Suitable inorganic acids are, for example, halogen acids, such as hydrochloric acid, sulfuric acid, or phosphoric acid.

### Taxanes

The term "taxane" relates to diterpenes produced by the plants of the genus Taxus (yews). They were first derived from natural sources, but some have been synthesized artificially. Taxanes have been used to produce various chemotherapy drugs and the primary mechanism of the taxane class of drugs is the disruption of microtubule function. Thus, taxanes are essentially mitotic inhibitors. The most well known taxane is paclitaxel which is better known under its registered trademark Taxol^{™}. In this formulation, paclitaxel is dissolved in Cremophor EL and ethanol, as a delivery agent. Another formulation, in which paclitaxel is bound to albumin, is paclitaxel sold under the trademark Abraxane^{™}. The term "taxane" includes taxane formulations such as liposomal paclitaxel and nab^{™}-paclitaxel taxane, taxane conjugates such as e.g. ANG-1005, and nano-based drug delivery systems for paclitaxel such as Genexol^{™} PM and Taclantis^{™} (Bevetex^{™}).

Thus, taxanes as referred herein usually comprise diterpenes produced by the plants of the genus Taxus (yews). In one embodiment the taxane is selected from the group consisting of paclitaxel, docetaxel, cabazitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), SB-T-1214, SB-T-1216, SB-T-121602, SB-T-12854, DHA-SB-T-1214, abeo-taxane 15a.2, cabazitaxel-7,10-d₆, docetaxel-f3-t-Boc, docetaxel-d₉-t-Boc, ANG-1005, cobalamin-paclitaxel, FK506-PEG₃-docetaxel, biotin-docetaxel (IDD-1010), biotin-SB-T-1214 (BLT-1), 4-ARM-PEG_{20K}-CM-Gly-d₉-DOC and 4-ARM-PEG_{20K}-BA-d₉-DOC, liposomal paclitaxel, nab^{™}-paclitaxel, Genexol^{™} PM and Taclantis^{™}.

In a preferred embodiment the taxane is selected from the group consisting of paclitaxel, docetaxel, cabazitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), liposomal paclitaxel, nab^{™}-paclitaxel, Genexol^{™} PM and Taclantis^{™}.

In a more preferred embodiment the taxane is selected from the group consisting of paclitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), liposomal paclitaxel, nab^{™}-paclitaxel, Genexol^{™} PM and Taclantis^{™}.

In an even more preferred embodiment the taxane is selected from the group consisting of paclitaxel, docosahexaenoic acid (DHA)-paclitaxel, poly(L-glutamic acid) PG-paclitaxel, ANG-1005, liposomal paclitaxel, nab^{™}-paclitaxel, Genexol^{™} PM and Taclantis^{™}.

In a particular embodiment the taxane is selected from the group consisting of paclitaxel, liposomal paclitaxel and nab^{™}-paclitaxel, and is preferably paclitaxel.

Paclitaxel (Taxol^{™}), a diterpenoid natural product, is described in Ojima et al., Expert Opin. Ther. Patents 2016, 26 (1), 1-20, and B. Kumar et al., Current Cancer Drug Targets 2017, 17 (4), 357-375, and is represented by the structural formula indicated below:

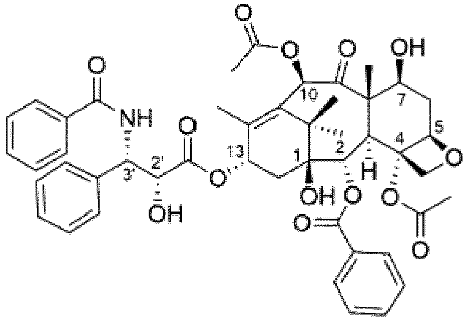

Docetaxel (Taxotere^{™}), a semi-synthetic analog of paclitaxel, is described in Ojima et al., Expert Opin. Ther. Patents 2016, 26 (1), 1-20 and B. Kumar et al., Current Cancer Drug Targets 2017, 17 (4), 357-375, and is represented by the structural formula indicated below:

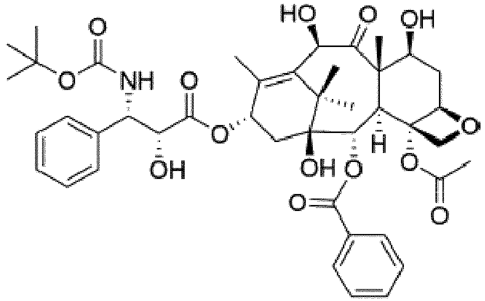

Cabazitaxel (Jevtana^{™}) is described in Ojima et al., Expert Opin. Ther. Patents 2016, 26 (1), 1-20, and B. Kumar et al., Current Cancer Drug Targets 2017, 17 (4), 357-375, and is represented by the structural formula indicated below:

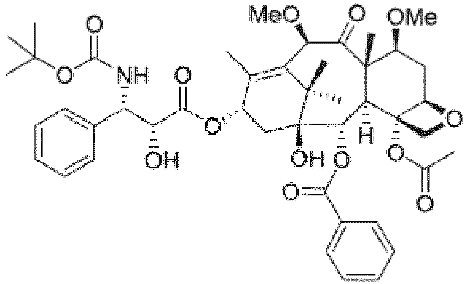

Larotaxel is described in Ojima et al., Expert Opin. Ther. Patents 2016, 26 (1), 1-20, and B. Kumar et al., Current Cancer Drug Targets 2017, 17 (4), 357-375, and is represented by the structural formula indicated below:

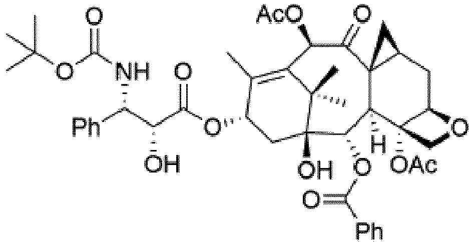

Ortataxel is described in Ojima et al., Expert Opin. Ther. Patents 2016, 26 (1), 1-20, B. Kumar et al., Current Cancer Drug Targets 2017, 17 (4), 357-375, and I. Ojima et al. J. Nat. Prod. 2018, 81, 703 - 721, and is represented by the structural formula indicated below:

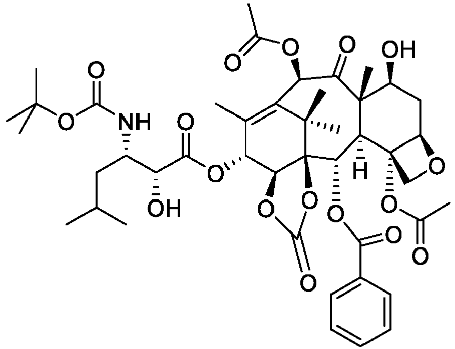

Tesetaxel is described in Ojima et al., Expert Opin. Ther. Patents 2016, 26 (1), 1-20, B. Kumar et al., Current Cancer Drug Targets 2017, 17 (4), 357-375, and M. Shionoya et al., Cancer Sci 2003, 94 (5), 459-466, and is represented by the structural formula indicated below: 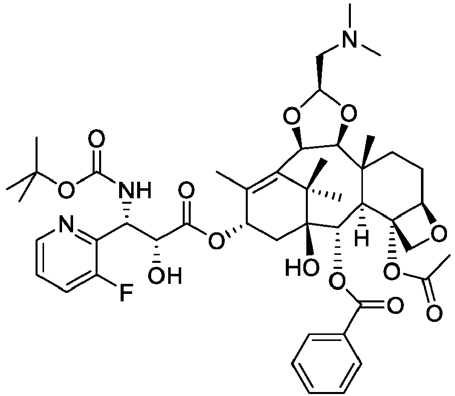 Milataxel is described in Ojima et al., Expert Opin. Ther. Patents 2016, 26 (1), 1-20, D. Sampath et al., Mol Cancer Ther 2003, 2, 873-884, and R. K. Ramanathan et al., Cancer Chemother Pharmacol 2008, 61, 453-458, and is represented by the structural formula indicated below:

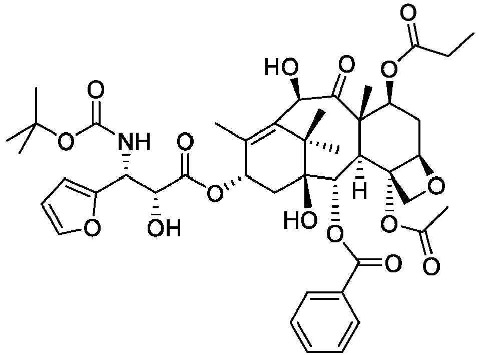

Docosahexaenoic acid (DHA)-paclitaxel (Taxoprexin^{™}) is described in Ojima et al., Expert Opin. Ther. Patents 2016, 26 (1), 1-20, and I. Ojima et al., Future Med. Chem. 2012, 4 (1), 33-50, and is represented by the structural formula indicated below:

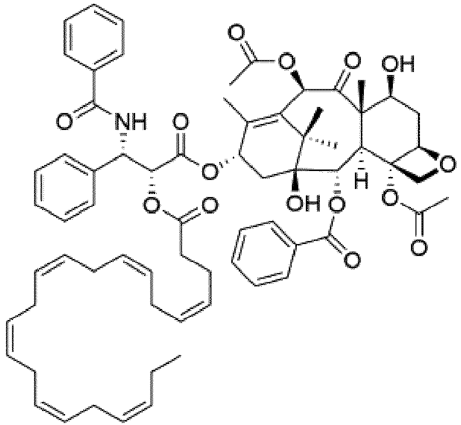

Poly(L-glutamic acid) PG-paclitaxel (Opaxio^{™}) is described in Ojima et al., Expert Opin. Ther. Patents 2016, 26 (1), 1-20 and J. W. Singer et al. in Macromolecular Anticancer Therapeutics, Cancer Drug Discovery and Development, L. H. Reddy, P. Couvreur (editors), Springer New York Dordrecht Heidelberg London, 2010, chapter 4, page 133 - 161, and is represented by the structural formula indicated below: 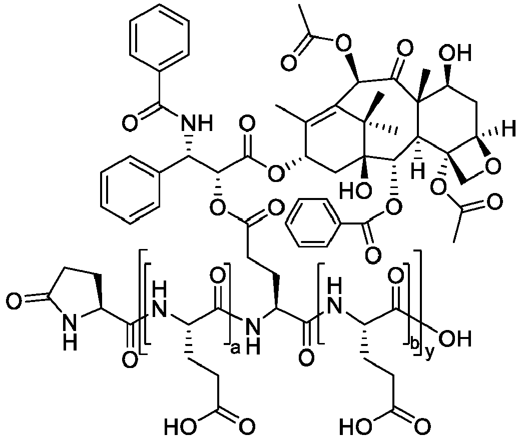 y, a, and b are independent variables

BMS-184476 (7-O-methylthiomethyl paclitaxel) is described in Ojima et al., Expert Opin. Ther. Patents 2016, 26 (1), 1-20, and T.J. Altstadt et al., J. Med. Chem. 2001, 44, 4577-4583, and is represented by the structural formula indicated below:

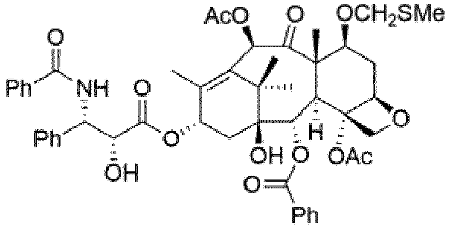

SB-T-1214 is described in Ojima et al., Expert Opin. Ther. Patents 2016, 26 (1), 1-20, I. Gut et al., Xenobiotica 2006, 36 (9), 772 - 792, and I. Ojima et al., J. Med. Chem. 2008, 51, 3203-3221, and is represented by the structural formula indicated below:

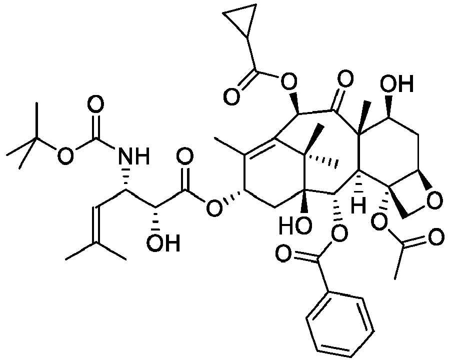

SB-T-1216 and SB-T-121602 are described in Ojima et al., Expert Opin. Ther. Patents 2016, 26 (1), 1-20, I. Gut et al., Xenobiotica 2006, 36 (9), 772 - 792, and I. Ojima et al. J. Nat. Prod. 2018, 81, 703-721, and is represented by the structural formula indicated below: 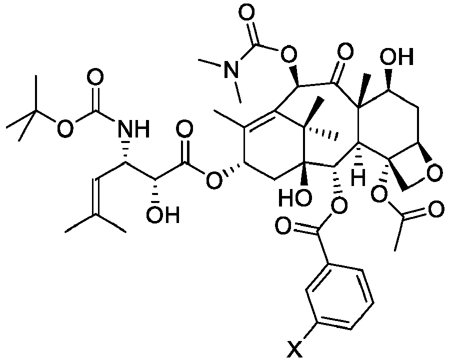
SB-T-1216: X = H
**SB-T-121602: X = CH₃**

SB-T-12854 is described in Ojima et al., Expert Opin. Ther. Patents 2016, 26 (1), 1-20, and I. Ojima et al. J. Nat. Prod. 2018, 81, 703-721, and is represented by the structural formula indicated below:

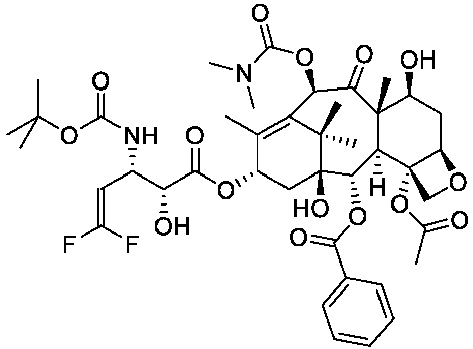

DHA-SB-T-1214 is described in Ojima et al., Expert Opin. Ther. Patents 2016, 26 (1), 1-20, and is represented by the structural formula indicated below:

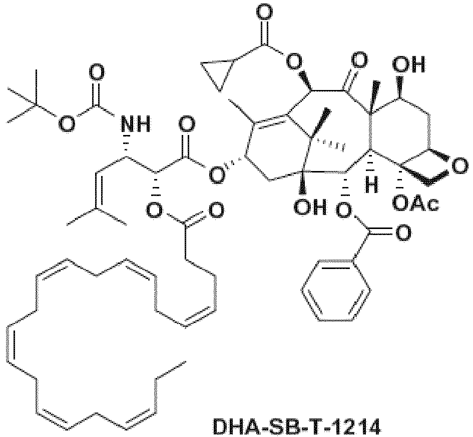

Abeo-taxane 15a.2 is described in Ojima et al., Expert Opin. Ther. Patents 2016, 26 (1), 1-20, and WO 2013/106029 A1, and is represented by the structural formula indicated below:

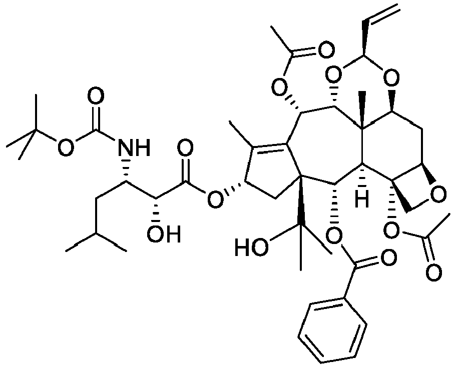

Cabazitaxel-7,10-d₆ is described in Ojima et al., Expert Opin. Ther. Patents 2016, 26 (1), 1-20, and is represented by the structural formula indicated below:

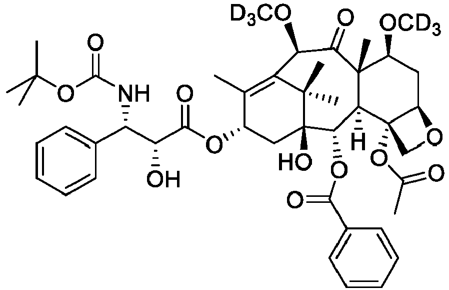

Docetaxel-f3-t-Boc is described in Ojima et al., Expert Opin. Ther. Patents 2016, 26 (1), 1-20, and is represented by the structural formula indicated below:

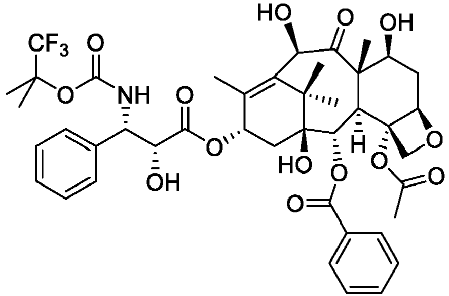

Docetaxel-dg-t-Boc is described in Ojima et al., Expert Opin. Ther. Patents 2016, 26 (1), 1-20, and is represented by the structural formula indicated below:

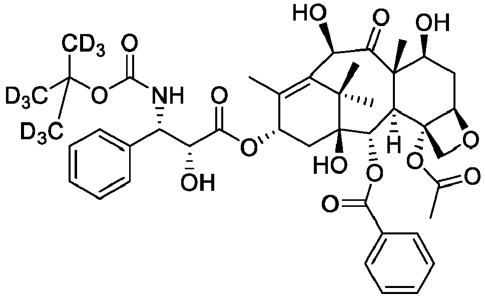

ANG-1005 is described in Ojima et al., Expert Opin. Ther. Patents 2016, 26 (1), 1-20, and WO 2010/121379 A1, and is represented by the structural formula indicated below:

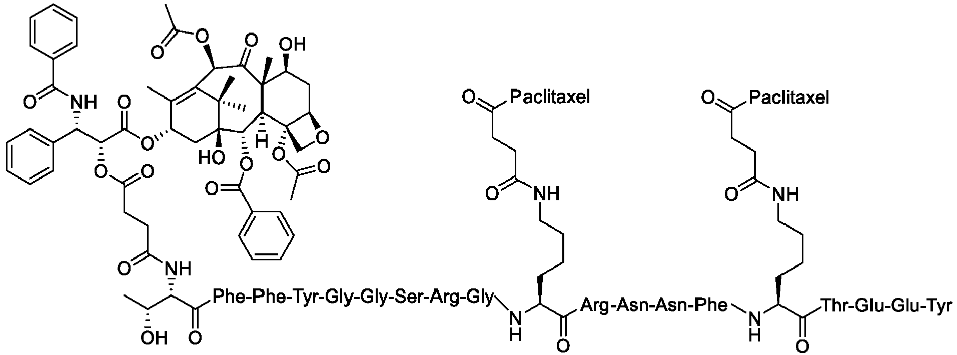

Cobalamin-paclitaxel is described in Ojima et al., Expert Opin. Ther. Patents 2016, 26 (1), 1-20, and WO 2008/115805 A2, and is represented by the structural formula indicated below:

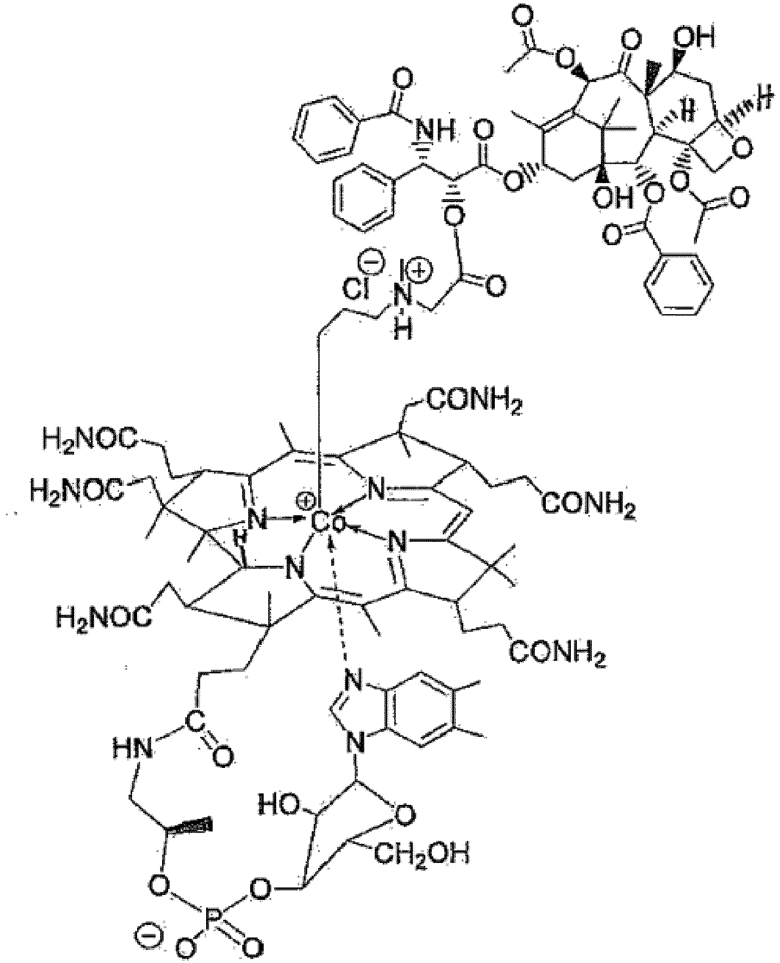

FK506-PEG₃-docetaxel is described in Ojima et al., Expert Opin. Ther. Patents 2016, 26 (1), 1-20, and WO 2011/130317 A2, and is represented by the structural formula indicated below:

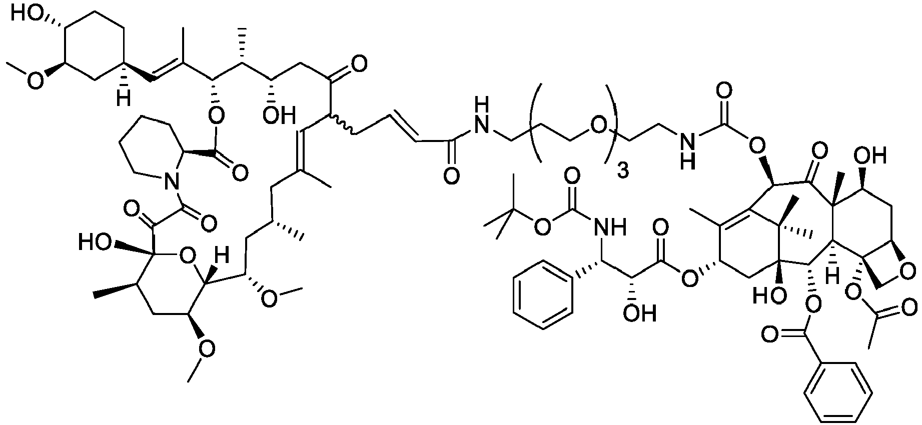

Biotin-docetaxel (IDD-1010) is described in Ojima et al., Expert Opin. Ther. Patents 2016, 26 (1), 1-20, and WO 2014/191989 A1, is represented by the structural formula indicated below:

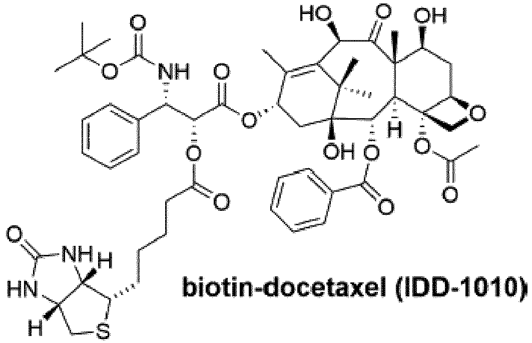

Biotin-SB-T-1214 (BLT-1) is described in Ojima et al., Expert Opin. Ther. Patents 2016, 26 (1), 1-20, and is represented by the structural formula indicated below:

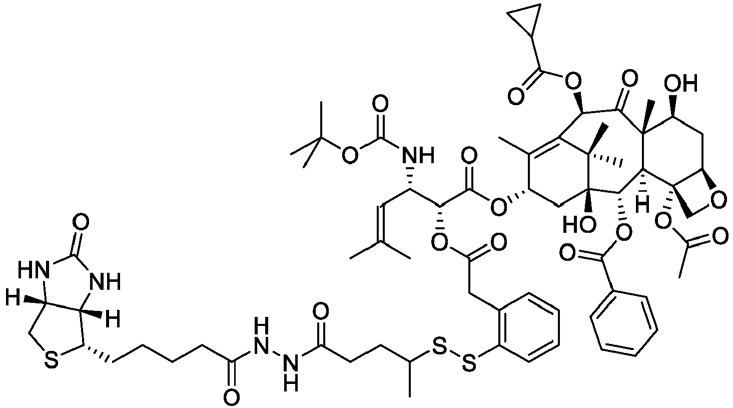

4-ARM-PEG_{20K}-CM-Gly-d₉-DOC is described in Ojima et al., Expert Opin. Ther. Patents 2016, 26 (1), 1-20, and WO 2012/088422 A1, and is represented by the structural formula indicated below:

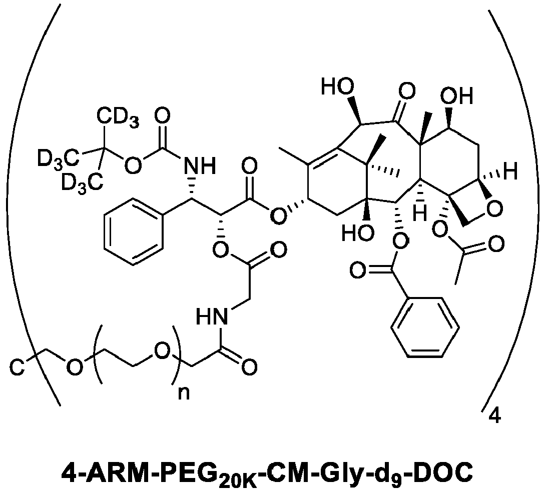

4-ARM-PEG_{20K}-BA-d₉-DOC is described in Ojima et al., Expert Opin. Ther. Patents 2016, 26 (1), 1-20, and WO 2012/088422 A1, and is represented by the structural formula indicated below:

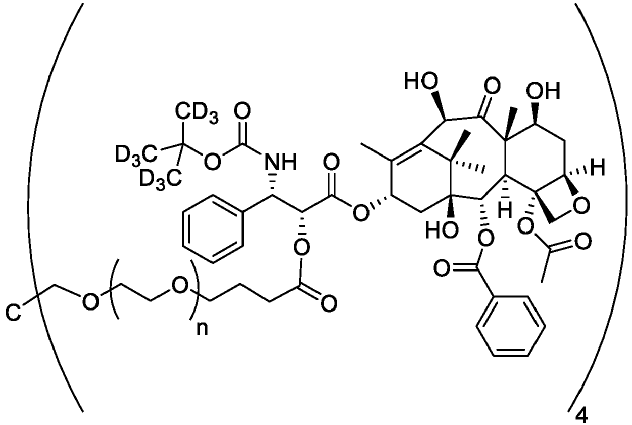

The abbreviations listed below have been used for certain substituents within the structures presented above: Me for methyl-, Ac for acetyl-, Ph for phenyl and Bz for benzoyl-.

The term "liposomal paclitaxel" as used herein refer to liposomal formulations of paclitaxel such as Lipusu^{™}, a liposomal paclitaxel formulation developed by Sike Pharmaceutical Co. Ltd., Nanjing, Jiangsu, P.R. China, which has been approved by the State Food and Drug Administration of China , and are described in S. Koudelka, J. Turanek, Journal of Controled Release 2012, 163, 322-334. The liposomal paclitaxel used in the present invention is preferably Lipusu^{™}.

Nab^{™}-paclitaxel (ABI-007; abraxane^{™}) is a nanoparticle albumin-bound paclitaxel. The human albumin-stabilized paclitaxel particles have an average size of ∼130 nm as described Ojima et al., Expert Opin. Ther. Patents 2016, 26 (1), 1-20.

Additional nano-based drug delivery systems for paclitaxel are Genexol^{™} PM and Taclantis^{™} (Bevetex^{™}) as described in Pi-Ling Cou et al., International Journal of Nanomedicine 2020, 15, 1731-1743). Genexol^{™} PM comprises polymeric micelle paclitaxel having an average particle size between 25 and 50 nm. The micelles comprise a monomethoxy poly(ethylene glycol)-block-poly(D,L-lactide) (mPEG-PDLLA) copolymer. Taclantis^{™} (Bevetex^{™}) is a paclitaxel injection concentrate for nanodispersion and based on a polyvinylpyrrolidone/paclitaxel self-assembly. The formulation of Taclantis^{™} (Bevetex^{™}) is cremophor free and human serum albumin free. The average particle size is between 100 and 110 nm.

### Pharmaceutical combinations

As outlined above, in a first aspect, the present invention provides a pharmaceutical combination comprising:
a) a peptidic CXCR4 inhibitor;
(b) a taxane; and
(c) optionally one or more pharmaceutically acceptable diluents, excipients or carriers.

A pharmaceutical combination according to the invention is for example a combined preparation or a pharmaceutical composition, for simultaneous, separate or sequential use.

The term "combined preparation" as used herein defines especially a "kit of parts" in the sense that said peptidic CXCR4 inhibitor and said taxane can be dosed independently, either in separate form e.g. as separate tablets or by use of different fixed combinations with distinguished amounts of the active ingredients. The ratio of the amount of peptidic CXCR4 inhibitor to the amount of taxane to be administered in the combined preparation can be varied, e.g. in order to cope with the needs of a patient sub-population to be treated or the needs of a single patient, which needs can be different due to age, sex, body weight, etc. of a patient. The individual parts of the combined preparation (kit of parts) can be administered simultaneously or sequentially, i.e. chronologically staggered, e.g. at different time points and with equal or different time intervals for any part of the kit of parts.

The term "pharmaceutical composition" refers to a fixed-dose combination (FDC) that includes the peptidic CXCR4 inhibitor and the taxane combined in a single dosage form, having a predetermined combination of respective dosages. In one embodiment, the pharmaceutical combination of the invention is a pharmaceutical composition and includes other medicinal or pharmaceutical agents, e.g., one or more pharmaceutically acceptable diluents, excipients or carriers.

The pharmaceutical combination further may be used as add-on therapy. As used herein, "add-on" or "add-on therapy" means an assemblage of reagents for use in therapy, the subject receiving the therapy begins a first treatment regimen of one or more reagents prior to beginning a second treatment regimen of one or more different reagents in addition to the first treatment regimen, so that not all of the reagents used in the therapy are started at the same time. For example, adding taxane therapy to a patient already receiving peptidic CXCR4 inhibitor therapy and vice versa.
In a preferred embodiment, the pharmaceutical combination according to the invention is a combined preparation.

The amount of the peptidic CXCR4 inhibitor and the taxane to be administered will vary depending upon factors such as the particular compound, disease condition and its severity, according to the particular circumstances surrounding the case, including, e.g., the specific peptidic CXCR4 inhibitor being administered, the route of administration, the condition being treated, the target area being treated, and the subject or host being treated.

In one embodiment, the invention provides a pharmaceutical combination comprising a peptidic CXCR4 inhibitor and a taxane, wherein said peptidic CXCR4 inhibitor and said taxane are present in a therapeutically effective amount.

The expression "effective amount" or "therapeutically effective amount" as used herein refers to an amount capable of invoking one or more of the following effects in a subject receiving the combination of the present invention: (i) increase of objective response rate (ORR); (ii) inhibition or arrest of tumor growth, including, reducing the rate of tumor growth or causing complete growth arrest; (iii) reduction in the number of tumor cells; (iv) reduction in tumor size; (v) reduction in tumor number; (vi) inhibition of metastasis (i.e. reduction, slowing down or complete stopping) of tumor cell infiltration into peripheral organs; (vii) enhancement of antitumor immune response, which may, but does not have to, result in the regression or elimination of the tumor; (viii) relief, to some extent, of one or more symptoms associated with cancer; (ix) increase in progression-free survival (PFS) and/or; overall survival (OS) of the subject receiving the combination.

Determination of a therapeutically effective amount is well within the capability of those skilled in the art, especially in light of the detailed disclosure provided herein. In some embodiments, a therapeutically effective amount of the peptidic CXCR4 inhibitor may (i) reduce the number of cancer cells; (ii) reduce tumor size; (iii) inhibit, retard, slow down to some extent, and preferably stop cancer cell infiltration into peripheral organs; (iv) inhibit (e.g., slow to some extent and preferably stop) tumor metastasis; (v) inhibit tumor growth; (vi) delay occurrence and/or recurrence of a tumor; and/or (vii) relieve to some extent one or more of the symptoms associated with the cancer. In various embodiments, the amount is sufficient to ameliorate, palliate, lessen, and/or delay one or more of symptoms of cancer. The therapeutically effective amount may vary depending on the subject, and disease or condition being treated, the weight and age of the subject, the severity of the disease or condition, and the manner of administering, which can readily be determined by one ordinary skilled in the art.

In a preferred embodiment, the invention provides a pharmaceutical combination comprising a peptidic CXCR4 inhibitor and a taxane, wherein said peptidic CXCR4 inhibitor and said taxane produce an additive therapeutic effect i.e. wherein said peptidic CXCR4 inhibitor and said taxane are present in an amount producing an additive therapeutic effect.

As used herein, the term "additive" means that the effect achieved with the pharmaceutical combinations of this invention is approximately the sum of the effects that result from using the agents, namely the peptidic CXCR4 inhibitor and the taxane, as a monotherapy.

In a further preferred embodiment, the invention provides a pharmaceutical combination comprising a peptidic CXCR4 inhibitor and a taxane, wherein said peptidic CXCR4 inhibitor and said taxane produce a synergistic therapeutic effect, i.e. wherein said peptidic CXCR4 inhibitor and said taxane are present in an amount producing a synergistic therapeutic effect.

As used herein, the term "synergistic" means that the effect achieved with the pharmaceutical combinations of this invention is greater than the sum of the effects that result from using the agents, namely the peptidic CXCR4 inhibitor and the taxane, as a monotherapy.

In one embodiment, the invention provides a pharmaceutical combination comprising a taxane and a peptidic CXCR4 inhibitor, wherein the amount of said peptidic CXCR4 inhibitor in the combination is is from about 0.3 to about 3500 mg, or from about 0.3 to about 2500 mg, or from about 0.3 to about 1600 mg, or from about 0.3 to about 1200 mg, or from about 0.3 to about 800 mg, or from about 1 to about 500 mg, preferably from about 1 to about 400 mg. Where said peptidic CXCR4 inhibitor is in the form of a pharmaceutically acceptable salt, the amounts of peptidic CXCR4 inhibitor provided herein are calculated on the basis of the respective free base.

In one embodiment, the invention provides a pharmaceutical combination comprising a taxane and a peptidic CXCR4 inhibitor, wherein the amount of said taxane in the combination is from about from about 0.2 to about 3500 mg, or from about 0.2 to about 1800 mg, or from about 0.2 to about 700 mg, or from about 1 to about 350 mg, or preferably from about 1 to about 200 mg.

In one embodiment, the invention provides a pharmaceutical combination comprising a taxane and a peptidic CXCR4 inhibitor, wherein the amount of said peptidic CXCR4 inhibitor in the combination is from about 0.3 to about 3500 mg, or from about 0.3 to about 2500 mg, or from about 0.3 to about 1600 mg, or from about 0.3 to about 1200 mg, or from about 0.3 to about 800 mg, or from about 1 to about 500 mg, preferably from about 1 to about 400 mg, and wherein the amount of said taxane in the combination is from about from about 0.2 to about 3500 mg, or from about 0.2 to about 1800 mg, or from about 0.2 to about 700 mg, or from about 1 to about 350 mg, or preferably from about 1 to about 200 mg.

In one embodiment, there is provided a pharmaceutical combination comprising:
(a) a peptidic CXCR4 inhibitor;
(b) a taxane; and
(c) optionally one or more pharmaceutically acceptable diluents, excipients or carriers,
wherein said peptidic CXCR4 inhibitor is a backbone cyclized peptidic compound, built up from 16 amino acid residues, or pharmaceutically acceptable salts thereof, of the formula

cyclo(-Tyr¹-His²-Xaa³-Cys⁴-Ser⁵-Ala⁶-Xaa⁷-Xaa⁸-Arg⁹-Tyr¹⁰-Cys¹¹-Tyr¹²-Gln¹³-Xaa¹⁴-Xaa¹⁵-Pro¹⁶-) (I),

in which
Xaa³ is Ala; Tyr; or Tyr(Me);
Xaa⁷ is ^{D}Pro; ^{D}Tyr; or ^{D}Tyr(Me);
Xaa⁸ is Dab; or Orn(iPr);
Xaa¹⁴ is Lys; or Lys(iPr);
Xaa¹⁵ is ^{D}Pro; or ^{D}Lys(iPr);
wherein
Tyr(Me) is (2*S*)-2-amino-3-(4-methoxyphenyl)-propanoic acid;
^{D}Tyr(Me) is (2*R*)-2-amino-3-(4-methoxyphenyl)-propanoic acid;
Dab is (2*S*)-2,4-diaminobutyric acid;
Orn(iPr) is (2*S*)-N^{ω}-isopropyl-2,5-diaminopentanoic acid;
Lys(iPr) is (2*S*)-N^{ω}-isopropyl-2,6-diaminohexanoic acid;
^{D}Lys(iPr) is (2*R*)-N^{ω}-isopropyl-2,6-diaminohexanoic acid;
wherein all of the amino acid residues, which are not explicitly designated as D-amino acid residues, are L-amino acid residues, and
wherein the compound of formula I has a disulfide bond between Cys⁴ and Cys¹¹, and
wherein the taxane is selected from the group consisting of paclitaxel, docetaxel, cabazitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), SB-T-1214, SB-T-1216, SB-T-121602, SB-T-12854, DHA-SB-T-1214, abeo-taxane 15a.2, cabazitaxel-7,10-d₆, docetaxel-f3-t-Boc, docetaxel-dg-t-Boc, ANG-1005, cobalamin-paclitaxel, FK506-PEG₃-docetaxel, biotin-docetaxel (IDD-1010), biotin-SB-T-1214 (BLT-1), 4-ARM-PEG_{20K}-CM-Gly-dg-DOC and 4-ARM-PEG_{20K}-BA-d₉-DOC, liposomal paclitaxel, nab^{™}-paclitaxel, Genexol^{™} PM and Taclantis^{™}.

In one embodiment, there is provided a pharmaceutical combination comprising:
(a) a peptidic CXCR4 inhibitor;
(b) a taxane; and
(c) optionally one or more pharmaceutically acceptable diluents, excipients or carriers,
wherein the peptidic CXCR4 inhibitor is a backbone cyclized peptidic compound, built up from 16 amino acid residues, or pharmaceutically acceptable salts thereof, selected from the group consisting of cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Lys(iPr)-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr-Dab-Arg-Tyr-Cys-Tyr-Gln-Lys(iPr)-^{D}Pro-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr-Cys-Ser-Ala-^{D}Tyr-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹,
or pharmaceutically acceptable salts thereof, and
wherein the taxane is selected from the group consisting of paclitaxel, docetaxel, cabazitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), SB-T-1214, SB-T-1216, SB-T-121602, SB-T-12854, DHA-SB-T-1214, abeo-taxane 15a.2, cabazitaxel-7,10-d₆, docetaxel-f3-t-Boc, docetaxel-dg-t-Boc, ANG-1005, cobalamin-paclitaxel, FK506-PEG₃-docetaxel, biotin-docetaxel (IDD-1010), biotin-SB-T-1214 (BLT-1), 4-ARM-PEG_{20K}-CM-Gly-dg-DOC and 4-ARM-PEG_{20K}-BA-d₉-DOC, liposomal paclitaxel, nab^{™}-paclitaxel, Genexol^{™} PM and Taclantis^{™}.

In one embodiment, there is provided a pharmaceutical combination comprising:
(a) a peptidic CXCR4 inhibitor;
(b) a taxane; and
(c) optionally one or more pharmaceutically acceptable diluents, excipients or carriers,
wherein the peptidic CXCR4 inhibitor is a backbone cyclized peptidic compound, built up from 16 amino acid residues, or pharmaceutically acceptable salts thereof, selected from the group consisting of cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Lys(iPr)-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr-Dab-Arg-Tyr-Cys-Tyr-Gln-Lys(iPr)-^{D}Pro-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr-Cys-Ser-Ala-^{D}Tyr-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹,
or pharmaceutically acceptable salts thereof, and
wherein the taxane is selected from the group consisting of paclitaxel, docetaxel, cabazitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), liposomal paclitaxel, nab^{™}-paclitaxel, Genexol^{™} PM and Taclantis^{™}.

In one embodiment, there is provided a pharmaceutical combination comprising:
(a) a peptidic CXCR4 inhibitor;
(b) a taxane; and
(c) optionally one or more pharmaceutically acceptable diluents, excipients or carriers,
wherein the peptidic CXCR4 inhibitor is a backbone cyclized peptidic compound, built up from 16 amino acid residues, or pharmaceutically acceptable salts thereof, selected from the group consisting of cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Lys(iPr)-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr-Dab-Arg-Tyr-Cys-Tyr-Gln-Lys(iPr)-^{D}Pro-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr-Cys-Ser-Ala-^{D}Tyr-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹,
or pharmaceutically acceptable salts thereof, and
wherein the taxane is selected from the group consisting of paclitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), liposomal paclitaxel, nab^{™}-paclitaxel, Genexol^{™} PM and Taclantis^{™}.

In one embodiment, there is provided a pharmaceutical combination comprising:
(a) a peptidic CXCR4 inhibitor;
(b) a taxane; and
(c) optionally one or more pharmaceutically acceptable diluents, excipients or carriers,
wherein the peptidic CXCR4 inhibitor is a backbone cyclized peptidic compound, built up from 16 amino acid residues, or pharmaceutically acceptable salts thereof, selected from the group consisting of cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His-Tyr-Cys-Ser-Ala-^{D}Pro-Dab-Arg-Tyr-Cys-Tyr-Gln-Lys-^{D}Pro-Pro-) having a disulfide bond between Cys⁴ and Cys¹¹, cyclo(-Tyr-His- Tyr -Cys-Ser-Ala-^{D}Pro-Orn(iPr)-Arg-Tyr-Cys-Tyr-Gln-Lys(iPr)-^{D}Lys(iPr)-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr-Dab-Arg-Tyr-Cys-Tyr-Gln-Lys(iPr)-^{D}Pro-Pro-) having a disulfide bond between Cys⁴ and Cys¹¹, cyclo(-Tyr-His-Tyr-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Pro-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Ala-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr-Cys-Ser-Ala-^{D}Tyr-Orn(iPr)-Arg-Tyr-Cys-Tyr-Gln-Lys(iPr)-^{D}Pro-Pro-) having a disulfide bond between Cys⁴ and Cys¹¹, cyclo(-Tyr-His-Tyr(Me)-Cys-Ser-Ala-^{D}Tyr(Me)-Orn(iPr)-Arg-Tyr-Cys-Tyr-Gln-Lys(iPr)-^{D}Pro-Pro-) having a disulfide bond between Cys⁴ and Cys¹¹,
or pharmaceutically acceptable salts thereof, and
wherein the taxane is selected from the group consisting of paclitaxel, docosahexaenoic acid (DHA)-paclitaxel, poly(L-glutamic acid) PG-paclitaxel, ANG-1005, liposomal paclitaxel, nab^{™}-paclitaxel, Genexol^{™} PM and Taclantis^{™}, more preferably selected from the group consisting of paclitaxel, liposomal paclitaxel, and nab^{™}-paclitaxel, and is even more preferably paclitaxel.

In one embodiment, there is provided a pharmaceutical combination comprising:
(a) a peptidic CXCR4 inhibitor;
(b) a taxane; and
(c) optionally one or more pharmaceutically acceptable diluents, excipients or carriers,
wherein said peptidic CXCR4 inhibitor is selected from the group consisting of cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, and cyclo(-Tyr-His-Tyr-Cys-Ser-Ala-^{D}Pro-Dab-Arg-Tyr-Cys-Tyr-Gln-Lys-^{D}Pro-Pro-) having a disulfide bond between Cys⁴ and Cys¹¹
or pharmaceutically acceptable salts thereof, and
wherein the taxane is selected from the group consisting of paclitaxel, docosahexaenoic acid (DHA)-paclitaxel, poly(L-glutamic acid) PG-paclitaxel, ANG-1005, liposomal paclitaxel, nab^{™}-paclitaxel, Genexol^{™} PM and Taclantis^{™}, more preferably selected from the group consisting of paclitaxel, liposomal paclitaxel and nab^{™}-paclitaxel, and is even more preferably paclitaxel.

In one embodiment, there is provided a pharmaceutical combination comprising:
(a) a peptidic CXCR4 inhibitor;
(b) a taxane; and
(c) optionally one or more pharmaceutically acceptable diluents, excipients or carriers,
wherein said peptidic CXCR4 inhibitor is cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, or pharmaceutically acceptable sals thereof, and
wherein the taxane is selected from the group consisting of paclitaxel, docosahexaenoic acid (DHA)-paclitaxel, poly(L-glutamic acid) PG-paclitaxel, ANG-1005, liposomal paclitaxel, nab^{™}-paclitaxel, Genexol^{™} PM and Taclantis^{™}, more preferably selected from the group consisting of paclitaxel, liposomal paclitaxel and nab^{™}-paclitaxel, and is even more preferably paclitaxel.

In a particular preferred embodiment there is provided a pharmaceutical combination comprising:
(a) a peptidic CXCR4 inhibitor;
(b) a taxane; and
(c) optionally one or more pharmaceutically acceptable diluents, excipients or carriers,
wherein said peptidic CXCR4 inhibitor is cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, or pharmaceutically acceptable sals thereof, and the taxane is paclitaxel.

### Formulations and modes of administration

The formulation and route of administration chosen may be tailored to the individual subject, the nature of the condition to be treated in the subject, and generally, the judgment of the attending practitioner.

The pharmaceutical compositions or combined preparations of the invention may be administered in either single or multiple doses by any of the accepted modes of administration of agents having similar utilities, including rectal, buccal, intranasal, transmucosal, transdermal, by intra-arterial injection, intravenously, intraperitoneally, parenterally, intramuscularly, subcutaneously, orally, topically, as e.g. an inhalant via pulmonary adminstration, or via an impregnated or coated device such as a stent, for example, or an artery-inserted cylindrical polymer.

One mode for administration is administration by injection, preferably intravenous administration by injection. The forms in which the peptidic CXCR4 inhibitor and/or taxane, may be incorporated for administration by injection include aqueous or oil suspensions, or emulsions, with sesame oil, corn oil, cottonseed oil, peanut oil, or castor oil, or chemical modified derivatives of the aforesaid oils thereof, as for example Cremophor EL, as well as elixirs, mannitol, dextrose, or a sterile aqueous solution, and similar pharmaceutical vehicles. Aqueous solutions in saline may also conventionally be used for injection, preferably physiologically compatible buffers such as Hank's solution, Ringer's solution, or physiological saline buffer are used as aqueous solutions. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating a compound according to the present disclosure in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze- drying techniques which yield a powder of the active ingredient plus any additional desired ingredient from a previously sterile-filtered solution thereof. In certain embodiments, for parenteral administration, sterile injectable solutions are prepared containing a therapeutically effective amount, e.g., 0.3 to 3500 mg, of the peptidic CXCR4 inhibitor and/or taxane. It will be understood, however, that the amount of the compound actually administered usually will be determined by a physician, in the light of the relevant circumstances, including the condition to be treated, the chosen route of administration, the actual compound administered and its relative activity, the age, weight, and response of the individual patient, the severity of the patient's symptoms, and the like.

A pharmaceutical combination according to the invention is, preferably, suitable for injection, e.g. subcutaneous, intravenous, intramuscular, intrathecal or intraperitoneal injection, more preferably suitable for intravenous injection, and usually comprises a therapeutically effective amount of the active ingredients and one or more suitable pharmaceutically acceptable diluent, excipient or carrier. Thus in a preferred embodiment the pharmaceutical combination is administered to the subject intravenously. i.e. the peptidic CXCR4 inhibitor and the taxane, respectively, are administered to the subject intravenously.

Pharmaceutical compositions or combined preparations in separate form comprising a peptidic CXCR4 inhibitor and taxane may be manufactured by means of conventional mixing, dissolving, granulating, coated tablet-making, levigating, emulsifying, encapsulating, entrapping or lyophilizing processes. Pharmaceutical compositions or combined preparations in separate form may be formulated in conventional manner using one or more physiologically acceptable carriers, diluents, excipients or auxilliaries which facilitate processing of the active ingredient into preparations which can be used pharmaceutically. Proper formulation depends upon the method of administration chosen.

For topical administration the peptidic CXCR4 inhibitor and/or taxane may be formulated as solutions, gels, ointments, creams, suspensions, etc. as are well-known in the art.

For transmucosal administration, penetrants appropriate to the barrier to be permeated are used in the formulation as known in the art.

For oral administration, the compounds can be readily formulated by combining the peptidic CXCR4 inhibitor and/or taxane with pharmaceutically acceptable carriers well known in the art. Such carriers enable the peptidic CXCR4 inhibitor and/or taxane to be formulated as tablets, pills, dragees, capsules, liquids, gels, syrups, slurries, suspensions etc., for oral ingestion by a patient to be treated. For oral formulations such as, for example, powders, capsules and tablets, suitable excipients include fillers such as sugars, e. g. lactose, sucrose, mannitol and sorbitol; cellulose preparations such as maize starch, wheat starch, rice starch, potato starch, gelatin, gum tragacanth, methyl cellulose, hydroxypropylmethyl cellulose, sodium carboxymethylcellulose; granulating agents; and binding agents. If desired, desintegrating agents may be added, such as cross-linked polyvinylpyrrolidones, agar, or alginic acid or a salt thereof, such as sodium alginate. If desired, solid dosage forms may be sugar-coated or enteric-coated using standard techniques. For oral liquid preparations such as, for example, suspensions, elixirs and solutions, suitable carriers, excipients or diluents include water, glycols, oils, alcohols, etc. In addition, flavoring agents, preservatives, coloring agents and the like may be added.

For buccal administration, the composition may take the form of tablets, lozenges, etc. formulated as usual.

For administration by inhalation, the peptidic CXCR4 inhibitor and/or taxane are conveniently delivered in form of an aeorosol spray from pressurized packs or a nebulizer, with the use of a suitable propellant, e.g. dichlorodifluoromethane, trichlorofluromethane, carbon dioxide or another suitable gas. In the case of a pressurized aerosol the dose unit may be determined by providing a valve to deliver a metered amount. Capsules and cartridges of e.g. gelatin for use in an inhaler or insufflator may be formulated containing a powder mix of the compounds of the invention and a suitable powder base such as lactose or starch.

The compounds may also be formulated in rectal or vaginal compositions such as suppositories together with appropriate suppository bases such as cocoa butter or other glycerides.

In addition to the formulations described previously, the peptidic CXCR4 inhibitor and/or taxane may also be formulated as depot preparations. Such long acting formulations may be administered by implantation (e.g. subcutaneously or intramuscularly) or by intramuscular injection. For the manufacture of such depot preparations the peptidic CXCR4 inhibitor and/or taxane may be formulated with suitable polymeric or hydrophobic materials (e.g. as an emulsion in an acceptable oil) or ion exchange resins, or as sparingly soluble salts.

In addition, other pharmaceutical delivery systems may be employed such as liposomes and emulsions well known in the art e.g. the taxanes of the presesent invention may be used as liposomal formulation such as e.g. the liposomal paclitaxel Lipusu^{™}. Certain organic solvents such as dimethylsulfoxide may also be employed. Additionally, the peptidic CXCR4 inhibitor and/or taxane may be delivered using a sustained-release system, such as semipermeable matrices of solid polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic agent, additional strategies for protein stabilization may be employed.

### Using the combinations of the invention to treat cancer

According to a second aspect the present invention provides a pharmaceutical combination as described herein, for use as a medicament.

According to a third aspect the present invention provides a pharmaceutical combination as described herein, for use in a method for the prevention, delay of progression or treatment of cancer in a subject, preferably for use in a method for the delay of progression or treatment of cancer in a subject, more preferably for use in a method for the treatment of cancer in a subject.

Also provided is the use of a pharmaceutical combination as described herein for the manufacture of a medicament for the prevention, delay of progression or treatment of cancer in a subject, preferably for the manufacture of a medicament for the delay of progression or treatment of cancer in a subject, more preferably for the manufacture of a medicament for the treatment of cancer in a subject.

Also provided is the use of a pharmaceutical combination as described herein for the prevention, delay of progression or treatment of cancer in a subject, preferably for the delay of progression or treatment of cancer in a subject, more preferably for the treatment of cancer in a subject.

Also provided is a method for the prevention, delay of progression or treatment of cancer in a subject, preferably a method for the delay of progression or treatment of cancer in a subject, more preferably a method for the treatment of cancer in a subject, comprising administering to said subject a pharmaceutical combination as described herein e.g. administering to said subject a therapeutically effective amount of a pharmaceutical combination as described herein.

As used herein, the term "prevention"/"preventing" e.g. preventive treatments comprise prophylactic treatments. In preventive applications, the pharmaceutical combination of the invention is administered to a subject suspected of having, or at risk for developing cancer.

As used herein, the term "delay of progression"/"delaying of progression" means increasing the time to appearance of a symptom of a cancer or a mark associated with a cancer or slowing the increase in severity of a symptom of a cancer. Further, "delay of progression" as used herein includes reversing or inhibition of disease progression. "Inhibition" of disease progression or disease complication in a subject means preventing or reducing the disease progression and/or disease complication in the subject.

The terms "treatment"/"treating" as used herein includes: (1) delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the development of the disease, or a relapse thereof in case of maintenance treatment, of at least one clinical or subclinical symptom thereof; and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.
In therapeutic applications, the pharmaceutical combination is usually administered to a subject such as a patient already suffering from cancer, in an amount sufficient to cure or at least partially arrest the symptoms of the disease. Amounts effective for this use will depend on the severity and course of the disease, previous therapy, the subject's health status and response to the drugs, and the judgment of the treating physician.
In the case wherein the subject's condition does not improve, the pharmaceutical combination of the invention may be administered chronically, which is, for an extended period of time, including throughout the duration of the subject's life in order to ameliorate or otherwise control or limit the symptoms of the subject's disease or condition.
In the case wherein the subject's status does improve, the pharmaceutical combination may be administered continuously; alternatively, the dose of drugs being administered may be temporarily reduced or temporarily suspended for a certain length of time (i.e., a "drug holiday").
Once improvement of the patient's condition has occurred, a maintenance dose of the pharmaceutical combination of the invention is administered if necessary. Subsequently, the dosage or the frequency of administration, or both, is optionally reduced, as a function of the symptoms, to a level at which the improved disease is retained.

In one embodiment of the invention, there is provided a pharmaceutical combination according to the invention, for use in a method for the prevention, delay of progression or treatment of cancer in a subject, preferably for use in a method for the delay of progression or treatment of a solid tumor in a subject, more preferably for use in a method for the treatment of cancer in a subject, wherein the cancer is a solid tumor selected from the group consisting of kaposi sarcoma, endometrial cancer, head and neck cancer, oesophageol cancer, breast cancers; lung cancer; pancreatic cancer, prostate cancer, colon cancer, ovarian cancer, and gastric cancer, even more preferably wherein the cancer is selected from the group consisting of breast cancer, metastatic breast cancer, and relapsed metastatic breast cancer, in particular selected from the group consisting of metastatic breast cancer and relapsed metastatic breast cancer, more praticular wherein the cancer is relapsed metastatic breast cancer.

Also provided is the use of a pharmaceutical combination as described herein for the manufacture of a medicament for the prevention, delay of progression or treatment of cancer in a subject, preferably for the manufacture of a medicament for the delay of progression or treatment of a solid tumor in a subject, more preferably for the manufacture of a medicament for the treatment of cancer wherein the cancer is a solid tumor selected from the group consisting of kaposi sarcoma, endometrial cancer, head and neck cancer, oesophageol cancer, breast cancers; lung cancer; pancreatic cancer, prostate cancer, colon cancer, ovarian cancer, and gastric cancer, even more preferably wherein the cancer is selected from the group consisting of breast cancer, metastatic breast cancer, and relapsed metastatic breast cancer, in particular selected from the group consisting of metastatic breast cancer and relapsed metastatic breast cancer, more particular wherein the cancer is relapsed metastatic breast cancer.

Also provided is the use of a pharmaceutical combination as described herein for the prevention, delay of progression or treatment of cancer in a subject, preferably for the delay of progression or treatment of a solid tumor in a subject, more preferably for use in a method for the treatment of cancer in a subject, wherein the cancer is a solid tumor selected from the group consisting of kaposi sarcoma, endometrial cancer, head and neck cancer, oesophageol cancer, breast cancers; lung cancer; pancreatic cancer, prostate cancer, colon cancer, ovarian cancer, and gastric cancer, even more preferably wherein the cancer is selected from the group consisting of breast cancer, metastatic breast cancer, and relapsed metastatic breast cancer, in particular selected from the group consisting of metastatic breast cancer and relapsed metastatic breast cancer, more praticular wherein the cancer is relapsed metastatic breast cancer.

Also provided is a method for the prevention, delay of progression or treatment of cancer in a subject, preferably a method for the delay of progression or treatment of cancer in a subject, more preferably a method for the treatment of a solid tumor in a subject, comprising administering to said subject a pharmaceutical combination as described herein e.g. administering to said subject a therapeutically effective amount of a pharmaceutical combination as described herein, wherein the cancer is a solid tumor selected from the group consisting of kaposi sarcoma, endometrial cancer, head and neck cancer, oesophageol cancer, breast cancers; lung cancer; pancreatic cancer, prostate cancer, colon cancer, ovarian cancer, and gastric cancer, even more preferably wherein the cancer is selected from the group consisting of breast cancer, metastatic breast cancer, and relapsed metastatic breast cancer, in particular selected from the group consisting of metastatic breast cancer and relapsed metastatic breast cancer, more praticular wherein the cancer is relapsed metastatic breast cancer.

In one embodiment the subject who has cancer is (i) refractory to at least one chemotherapy treatment, or (ii) is in relapse after treatment with chemotherapy, or a combination thereof. In some embodiments, the subject is refractory to at least two, at least three, or at least four anti-cancer therapy (including, for example, standard or experimental chemotherapies).
A subject who is refractory to at least one anti-cancer therapy and/or is in relapse after treatment with at least one anti-cancer therapy, as described above, may have undergone one or more prior therapies. In some embodiments, such subjects have undergone one, two, three, or four, or five, or at least one, at least two, at least three, at least four, or at least five, or between one and ten, between one and nine, between one and eight, between one and seven, between one and six, between one and five, or between one and four, or between one and three, between four and six or between seven and ten anti-cancer therapies prior to treatment using the methods described herein (e.g., prior to the administration of a peptidic CXCR4 inhibitor and a taxane).

### Dosing regimen

The dosing regimen of the peptidic CXCR4 inhibitor in combination with a taxane, in the methods provided herein may vary depending upon the indication, route of administration, and severity of the condition, for example. Depending on the route of administration, a suitable dose can be calculated according to body weight, body surface area, or organ size. The final dosing regimen can be determined by the attending physician in view of good medical practice, considering various factors that modify the action of drugs, e.g., the specific activity of the compound, the identity and severity of the disease state, the responsiveness of the patient, the age, condition, body weight, sex, and diet of the patient, and the severity of any infection. Additional factors that can be taken into account include time and frequency of administration, drug combinations, reaction sensitivities, and tolerance/response to therapy. Further refinement of the doses appropriate for treatment involving any of the formulations mentioned herein is done routinely by the skilled practitioner without undue experimentation, especially in light of the dosing information and assays disclosed, as well as the pharmacokinetic data observed in human clinical trials. Appropriate doses can be ascertained through use of established assays for determining concentration of the agent in a body fluid or other sample together with dose response data.

The amount, e.g. the therapeutically effective amount of the peptidic CXCR4 inhibitor may be provided in a single dose or multiple doses to achieve the desired treatment endpoint.

The frequency of dosing will depend on the pharmacokinetic parameters of the compound administered, the route of administration, and the particular disease treated. The dose and frequency of dosing may also depend on pharmacokinetic and pharmacodynamic, as well as toxicity and therapeutic efficiency data. For example, pharmacokinetic and pharmacodynamic information about a peptidic CXCR4 inhibitor and a taxane, can be collected through preclinical in vitro and in vivo studies, later confirmed in humans during the course of clinical trials. Thus, for the peptidic CXCR4 inhibitor and the taxane, used in the methods provided herein, a therapeutically effective dose can be estimated initially from biochemical and/or cell-based assays. Then, dosage can be formulated in animal models to achieve a desirable circulating concentration range. As human studies are conducted further information will emerge regarding the appropriate dosage levels and duration of treatment for various diseases and conditions.

Toxicity and therapeutic efficacy of a peptidic CXCR4 inhibitor and a taxane, can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the "therapeutic index", which typically is expressed as the ratio LD50/ED50. Compounds that exhibit large therapeutic indices, i.e. the toxic doses are substantially higher than the effective doses, are preferred. The data obtained from such cell culture assays and additional animal studies can be used in formulating a range of dosage for human use. The doses of such compounds lies preferably within a range of circulating concentrations that include the ED50 with little or no toxicity.

A peptidic CXCR4 inhibitor and a taxane, may be administered to the subject (e.g. a human) within minutes or hours. In some embodiments, a peptidic CXCR4 inhibitor and/or a taxane, may be administered to the subject (e.g. a human) over about 1 to about 240 minutes, over about 1 to about 180 minutes, or over about 5 to about 150 minutes, or over about 5 to about 120 minutes, or over about 1 to 60 minutes, or over about 1 to 10 minutes, or over about 5 minutes.

An exemplary treatment regime entails administration once daily, twice daily, three times daily, every day, every second day, every third day, every fourth day, every fifth day, every sixth day, twice per week, once per week. The combination of the invention is usually administered on multiple occasions. Intervals between single dosages can be, for example, less than a day, a day, two days, three days, four days, five days, six days or a week. The combination of the invention may be given as a continous uninterrupted treatment. The combination of the invention may also be given in a regime in which the subject receives cycles of treatment (administration cycles) interrupted by a drug holiday or period of non-treatment. Thus, the combination of the invention may be administered according to the selected intervals above for a continuous period of one week or a part thereof, for two weeks, for three weeks for four weeks, for five weeks or for six weeks and then stopped for a period of one week, or a part thereof, for two weeks, for three weeks, for four weeks, for five weeks, or for six weeks or for even more weeks. The combination of the treatment interval and the non-treatment interval is called a cycle. The cycle may be repeated one or more times. Two or more different cycles may be used in combination for repeating the treatment one or more times. The administration of the pharmaceutical combination according to the invention may start with a run-in cycle.

Exemplary doses of the peptidic CXCR4 inhibitor for a subject, preferably for a human subject, may be from about 0.1 to about 700 mg/kg, or from about 0.1 to about 500 mg/kg, or from about 0.1 to about 250 mg kg; or from about 0.1 to about 200 mg/kg, or from about 0.1 to about 150 mg/kg, or from about 0.1 to about 100 mg/kg, or from about 0.1 to about 75 mg/kg, or from about 0.1 to about 50 mg/kg, or from about 0.3 to about 50 mg/kg, or from about 1 to about 50 mg/kg, or from about 5 to about 50 mg/kg, or from about 5 to about 35 mg/kg.

Exemplary doses of taxane, for a subject, preferably for a human subject, may be from about 0.1 to about 50 mg/kg or from about 0.1 to about 25 mg/kg or from about 0.1 to about 10 mg/kg or from about 0.1 to about 5 mg/kg or from about 0.5 to about 5 mg/kg.

In one embodiment, the invention provides a pharmaceutical combination comprising a peptidic CXCR4 inhibitor and taxane, wherein the amount of said peptidic CXCR4 inhibitor in the combination from about 0.1 to about 700 mg/kg, or from about 0.1 to about 500 mg/kg, or from about 0.1 to about 250 mg kg; or from about 0.1 to about 200 mg/kg, or from about 0.1 to about 150 mg/kg, or from about 0.1 to about 100 mg/kg, or from about 0.1 to about 75 mg/kg, or from about 0.1 to about 50 mg/kg, or from about 0.3 to about 50 mg/kg, or from about 1 to about 50 mg/kg, or from about 5 to about 50 mg/kg, or from about 5 to about 35 mg/kg and wherein the amount of said taxane in the combination is from about 0.1 to about 50 mg/kg or from about 0.1 to about 25 mg/kg or from about 0.1 to about 10 mg/kg or from about 0.1 to about 5 mg/kg or from about 0.5 to about 5 mg/kg.

In a preferred embodiment, the invention provides a pharmaceutical combination comprising a peptidic CXCR4 inhibitor and taxane, wherein the amount of said peptidic CXCR4 inhibitor in the combination is from about 0.1 to about 150 mg/kg; and wherein the amount of said taxane in the combination is from about 0.1 to about 25 mg/kg.

In one embodiment, the invention provides a pharmaceutical combination comprising a peptidic CXCR4 inhibitor and taxane, wherein the peptidic CXCR4 inhibitor is administered to the subject at a dose from about 0.1 to about 700 mg/kg, or from about 0.1 to about 500 mg/kg, or from about 0.1 to about 250 mg kg; or from about 0.1 to about 200 mg/kg, or from about 0.1 to about 150 mg/kg, or from about 0.1 to about 100 mg/kg, or from about 0.1 to about 75 mg/kg, or from about 0.1 to about 50 mg/kg, or from about 0.3 to about 50 mg/kg, or from about 1 to about 50 mg/kg, or from about 5 to about 50 mg/kg, or from about 5 to about 35 mg/kg.

In one embodiment, the invention provides a pharmaceutical combination comprising a peptidic CXCR4 inhibitor and taxane, wherein taxane is administered to the subject at a dose from about 0.1 to about 50 mg/kg or from about 0.1 to about 25 mg/kg or from about 0.1 to about 10 mg/kg or from about 0.1 to about 5 mg/kg or from about 0.5 to about 5 mg/kg.

### Additional combination therapies

Provided herein are also methods of treatment in which the peptidic CXCR4 inhibitor in combination with a taxane, is administered to a subject (e.g. a human) in additional combination with one or more additional therapies. Thus, in some embodiments, the method for treating cancer in a subject (e.g. a human), comprises administering to the subject a therapeutically effective amount of a peptidic CXCR4 inhibitor and a taxane, together with one or more additional therapies, which can be useful for treating the cancer. The one or more additional therapies may involve the administration of one or more therapeutic agents, preferably therapeutic anti-cancer agents.

### Kit of parts

A pharmaceutical combination e.g. a combined preparation (including, for example, formulations and unit dosages) comprising the peptidic CXCR4 inhibitor and the taxane, can be prepared and placed in an appropriate container, and labeled for treatment of an indicated condition. Kits of parts also are contemplated. For example, a kit can comprise unit dosage forms of the peptidic CXCR4 inhibitor and the taxane, and a package insert containing instructions for use of the composition in treatment of a medical condition. In some embodiments, the kits comprises a unit dosage form of peptidic CXCR4 inhibitor and/or a taxane. The instructions for use in the kit may be for treating a cancer.

Thus in a fourth aspect the present invention provides a kit of parts comprising a first container, a second container and a package insert, wherein the first container comprises at least one dose of a medicament comprising a peptidic CXCR4 inhibitor; the second container comprises at least one dose of a medicament comprising a taxane, and the package insert comprises optionally instructions for treating a subject for cancer using the medicaments. The cancer the instructions relate are usually the cancers as described supra.

### Examples

The present examples are intended to illustrate the present invention without restricting it.

### Example 1

Cell line-derived xenografts CDX were for a long time the gold-standard mouse models used for the research and testing of anti-cancer therapies. Human tumor samples were cultured as cell lines in vitro and then implanted into immunocompromised mice to test the efficacy of anti-tumor compounds in vivo. Unfortunately, CDXs and syngeneic models have failed to predict human efficacy for most therapies targeted to cancer-driving proteins as evidenced by the low FDA approval rate for targeted therapeutics. Current standard preclinical practice by syngeneic models and CDXs inadequately addresses complex challenges to successful treatment, such as host immune responses, cancer heterogeneity and drug resistance. Consequently, the system cannot be used to optimize a multitude of variables known to influence therapeutic outcomes, such as combinatorial therapies, dosing schedules and drug delivery methods. Relative to CDX models, immunocompromised mice bearing subcutaneous surgically-derived clinical tumor samples (PDX models) are better aligned with human disease, since intact tissue that preserves tumor architecture is transferred directly to recipient mice and not compromised by in vitro adaptation. Humanized mice PDX model studies represent the most relevant, translational approach to test novel immunotherapies. Human tumor PDX tissue is implanted in mice that feature an intact humanized immune system, better representing the clinic. CD34+ humanized PDX mice models are ideal for long-term oncology studies as they involve stable engraftment of huCD34+ hematopoietic stem cells (HSC), and produce multi-lineage human immune cells that are present up to nine months later. (doi: 10.1016/j.cell.2015.08.068).

The study was designed to determine the response of the breast cancer PDX Ma15169 to treatment with Paclitaxel single agent, Balixafortide single agent and the combination of both in humanized NOG-EXL mice. Humanized NOG-EXL mice bearing the breast cancer PDX Ma15169 were treated with Paclitaxel and Balixafortide according to Table 1. Ma15169 is a patient derived xenograft (PDX) that was from a 67 years old patient with an invasive ductal carcinoma (ypT1a ypNla R0 G3 L1 V1). The patient tumour was ER/PR as well as Her-2 negative. The tumour material was expanded in 9 donor mice (NOG, Taconic; https://www.taconic.com/mouse-model/ciea-nog-mouse) and fragments of about 10mm3 were each implanted subcutaneously into the flank of 50 humanized NOG-EXL mice; at this time, the passage number of Ma15169 was #4.

**Table 1: Course of events during the study**

| **Event** | **Day of the study** |
|---|---|
| Arrival of 50 NOGs at EPO* | |
| Radiation | Day 0 |
| CD34+ cell inoculation | Day 1 |
| FACS I | Day 46 |
| FACS II | Day 84 |
| FACS III | Day 115 |
| PDX Ma15169 implantation | Day 122 |
| First treatment | Day 154 |
| Last treatment | Day 182 |
| Last TV measurement | Day 183 |
| FACS IV | Day 183 |
| Termination | Day 183 |

| | |
|---|---|
| *: EPO: Experimental Pharmacology & Oncology Berlin-Buch GmbH, Berlin-Buch, Germany | |

### Humanization using CD34+ cells

Female NOG-EXL mice with an age of about 4 weeks were used for the constitution of human immune cells. 24 hours before CD34+ cell inoculation mice were irradiated with 1Gy. Human CD34+ cells were isolated from fresh cord blood within the laboratories of EPO (Experimental Pharmacology & Oncology Berlin-Buch GmbH, Berlin-Buch, Germany) and preserved in liquid nitrogen until use. For the study MV16883 a pool of 5 different donors (CB AG 101, CB AG 97, CB KR 104, CB AG 106, CB AG 107) prepared. After thawing the cells were washed with PBS, pooled and washed again followed by Trypan blue staining and cell counting using Neubauer chamber. A total of 5x104 vital cells were resuspended in 200µl PBS and injected intravenously into the tail vain of all mice.

Determination of human immune cells by FACS was performed at days 46, 84 and 115 after cell inoculation (leucocytes CD45+, B cells CD19+, T cells CD3+, NK cells CD56+/CD16+ and monocytes CD14+; Human Immunophenotyping 8-color-Kit (Miltenyi)). The mice were weighed weekly and monitored for general health two times weekly.

### Tumor implantation

The PDX Ma15169 is maintained by EPO (Experimental Pharmacology & Oncology Berlin-Buch GmbH, Berlin-Buch, Germany) as *in vivo* passage; the passage number for this study was #4. Donor mice (female NOG; Taconic) with tumors of about 1cm3 were used for the preparation of this study MV16883. The retrieved tumors were solid and free of necrotic or fibrotic tissue (macroscopic assessment).

After removal of the tumor tissue from donor mice, the tumors were cut into fragments (2-3 mm diameter - approximately 10mm3) and placed in cooled RPMI 1640 culture medium until s.c. implantation within 30 minutes. The humanized NOG-EXL mice were anaesthetized with Etomidate and received a small incision in the skin of the left flank. The tumor fragments (one fragment/mouse) were implanted with tweezers and the incision closed with a suture clip (#310417, Covetrus GmbH). The clips were removed after seven days and mice controlled two times weekly by means of palpation. The implantation of tumor fragments was performed 122 days after the inoculation of CD34+ cells.

### Treatment

Tumor bearing mice were stratified into 4 treatment groups according to their tumor volumes at day 154 post CD34+ cell inoculation (32 days after tumor implantation). Due to heterogeneous tumor growth and lack of engraftment 34 mice with established tumors entered the efficacy study. The individual tumor volumes of these mice ranged from 0.110 to 0.352 cm3. During stratification the animals were individually marked by earmarks.

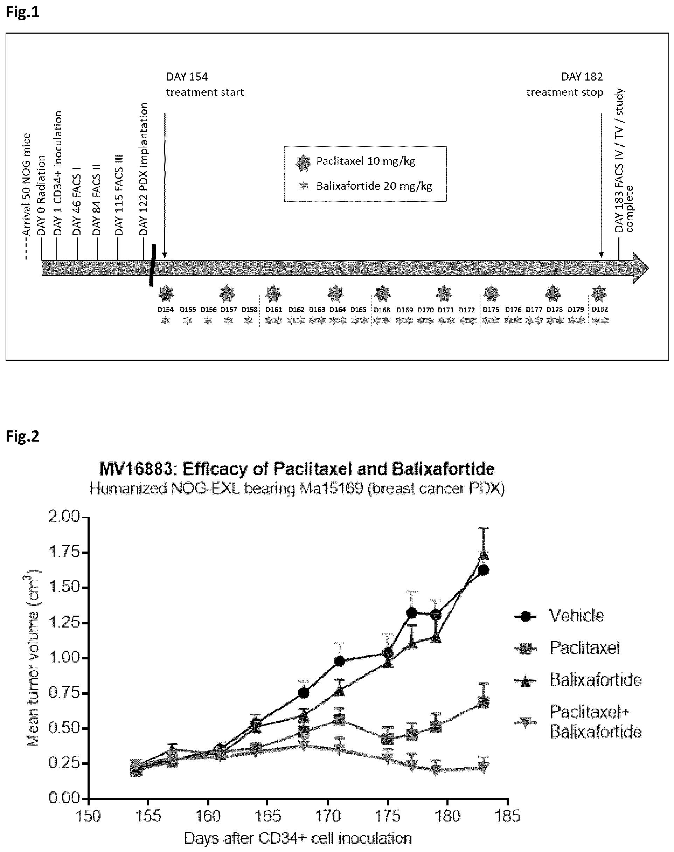

Paclitaxel (Taxomedac^{™} comprising paclictaxel, citric acid, ethanol and PEG (30-50) castor oil) was given i.v. at a dose of 10 mg/kg twice weekly. Balixafortide was given s.c. at a dose of 20 mg/kg once daily for the first 5 days and twice daily for 5 days followed by treatment breaks of 2 days until the end of treatment period (**Fig. 1**). Total treatment duration was 29 days. Successful engraftment of human CD34+ cells was checked by flow cytometry (FACS). Changes in the human immune cell population in blood were followed-up at the end of the study by FACS analysis. Primary read-out in this study were changes in the tumor volume calculated according to V=(length x (with)²)/2. At each measurement day the median and mean volumes per group and treated-to-control (T/C) were calculated.

### Results

Treatment with Paclitaxel resulted in significant tumor growth inhibition of (T/C 42%, day 183, p<0.05; stable disease). Balixafortide monotherapy inhibited tumor growth marginally. The optimum T/C value of Balixafortide was 79 % (p > 0.05) found at day 168. At the last day of the study, the T/C value increased to 107 % (progressive disease). T/C values > 50 % are rated as progressive disease according to RECIST criteria. The combination of Paclitaxel with Balixafortide improved the therapeutic effect significantly and in a synergistic manner. At the last day of the study, the T/C value of the combination group was 13 % compared to 42% and 107% of Paclitaxel and Balixafortide alone, respectively. According to RECIST criteria, a T/C value of 13% is rated as "partial remission". The improved therapeutic effect in the combination group was first time observed at day 168,14 days after the first dose of Balixafortide, and remained until the end of treatment period (**Fig. 2**).

## Claims

1. A pharmaceutical combination comprising:
(a) a peptidic CXCR4 inhibitor;
(b) a taxane; and
(c) optionally one or more pharmaceutically acceptable diluents, excipients or carriers.

2. A pharmaceutical combination according to claim 1, wherein said peptidic CXCR4 inhibitor is a backbone cyclized peptidic compound, built up from 16 amino acid residues, or pharmaceutically acceptable salts thereof, of the formula
cyclo(-Tyr¹-His²-Xaa³-Cys⁴-Ser⁵-Ala⁶-Xaa⁷-Xaa⁸-Arg⁹-Tyr¹⁰-Cys¹¹-Tyr¹²-Gln¹³-Xaa¹⁴-Xaa¹⁵-Pro¹⁶-) (I),
in which
Xaa³ is Ala; Tyr; or Tyr(Me);
Xaa⁷ is ^{D}Pro; ^{D}Tyr; or ^{D}Tyr(Me);
Xaa⁸ is Dab; or Orn(iPr);
Xaa¹⁴ is Lys; or Lys(iPr);
Xaa¹⁵ is ^{D}Pro; or ^{D}Lys(iPr);
wherein Tyr(Me) is (2*S*)-2-amino-3-(4-methoxyphenyl)-propanoic acid;
^{D}Tyr(Me) is (2*R*)-2-amino-3-(4-methoxyphenyl)-propanoic acid;
Dab is (2S)-2,4-diaminobutyric acid;
Orn(iPr) is (2*S*)-N^{ω}-isopropyl-2,5-diaminopentanoic acid;
Lys(iPr) is (2*S*)-N^{ω}-isopropyl-2,6-diaminohexanoic acid;
^{D}Lys(iPr) is (2*R*)-N^{ω}-isopropyl-2,6-diaminohexanoic acid;
wherein all of the amino acid residues, which are not explicitly designated as D-amino acid residues, are L-amino acid residues, and
wherein the compound of formula I has a disulfide bond between Cys⁴ and Cys¹¹.

3. A pharmaceutical combination according to claim 1, wherein said peptidic CXCR4 inhibitor is selected from the group consisting of cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹, and cyclo(-Tyr-His-Tyr-Cys-Ser-Ala-^{D}Pro-Dab-Arg-Tyr-Cys-Tyr-Gln-Lys-^{D}Pro-Pro-) having a disulfide bond between Cys⁴ and Cys¹¹,
or pharmaceutically acceptable salts thereof.

4. A pharmaceutical combination according to claim 1, wherein said peptidic CXCR4 inhibitor is cyclo(-Tyr-His-Ala-Cys-Ser-Ala-^{D}Pro-Dab-Arg-Tyr-Cys-Tyr-Gln-Lys-^{D}Pro-Pro-) having a disulfide bond between Cys⁴ and Cys¹¹,
or pharmaceutically acceptable sals thereof.

5. A pharmaceutical combination according to any one of claims 1 to 4, wherein the taxane is selected from the group consisting of paclitaxel, docetaxel, cabazitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), SB-T-1214, SB-T-1216, SB-T-121602, SB-T-12854, DHA-SB-T-1214, abeo-taxane 15a.2, cabazitaxel-7,10-d₆, docetaxel-f3-t-Boc, docetaxel-dg-t-Boc, ANG-1005, cobalamin-paclitaxel, FK506-PEG₃-docetaxel, biotin-docetaxel (IDD-1010), biotin-SB-T-1214 (BLT-1), 4-ARM-PEG_{20K}-CM-Gly-d₉-DOC and 4-ARM-PEG_{20K}-BA-d₉-DOC, liposomal paclitaxel, nab^{™}-paclitaxel, Genexol^{™} PM and Taclantis^{™}.

6. A pharmaceutical combination according to any one of claims 1 to 4, wherein the taxane is selected from the group consisting of paclitaxel, docetaxel, cabazitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), liposomal paclitaxel, nab^{™}-paclitaxel, Genexol^{™} PM and Taclantis^{™}.

7. A pharmaceutical combination according to any one of claims 1 to 4, wherein the taxane is selected from the group consisting of paclitaxel, larotaxel, ortataxel, tesetaxel, milataxel, docosahexaenoic acid (DHA)-paclitaxel, poly(L-glutamic acid) PG-paclitaxel, BMS-184476 (7-O-methylthiomethyl paclitaxel), liposomal paclitaxel, nab^{™}-paclitaxel, Genexol^{™} PM and Taclantis^{™}.

8. A pharmaceutical combination according to any one of claims 1 to 4, wherein the taxane is selected from the group consisting of paclitaxel, docosahexaenoic acid (DHA)-paclitaxel, poly(L-glutamic acid) PG-paclitaxel, ANG-1005, liposomal paclitaxel, nab^{™}-paclitaxel, Genexol^{™} PM and Taclantis^{™}.

9. A pharmaceutical combination according to any one of claims 1 to 4, wherein the taxane is selected from the group consisting of paclitaxel, liposomal paclitaxel and nab^{™}-paclitaxel, and is preferably paclitaxel.

10. A pharmaceutical combination according to any one of claims 1 to 9, for use as a medicament.

11. A pharmaceutical combination according to any one of claims 1 to 9 for use in a method for the prevention, delay of progression or treatment of cancer in a subject.

12. A pharmaceutical combination for use according to claim 11, wherein the cancer is a solid tumor.

13. A pharmaceutical combination for use according to claim 12, wherein the solid tumor is selected from the group consisting of kaposi sarcoma, endometrial cancer, head and neck cancer, oesophageol cancer, breast cancers, lung cancer, pancreatic cancer, prostate cancer, colon cancer, ovarian cancer, and gastric cancer.

14. A pharmaceutical combination for use according to claim 11, wherein the cancer is selected from the group consisting of breast cancer, metastatic breast cancer, and relapsed metastatic breast cancer.

15. A kit of parts comprising a first container, a second container and a package insert, wherein the first container comprises at least one dose of a medicament comprising a peptidic CXCR4 inhibitor; the second container comprises at least one dose of a medicament comprising a taxane, and the package insert comprises optionally instructions for treating a subject for cancer using the medicaments.
